# EUROPEAN PATENT APPLICATION

(11) **EP 3 767 341 A1**
(43) Date of publication of application: **20.01.2021**
(21) Application number: 18909691.0
(22) Date of filing: 31.08.2018
(51) Int. Cl.: G01T 1/20, A61B 6/00

(54) **RADIATION DETECTION PANEL, RADIATION DETECTOR, AND METHOD FOR MANUFACTURING RADIATION DETECTION PANEL**

(30) Priority: 12.03.2018 JP 2018044610
(71) Applicant: Canon Electron Tubes & Devices Co., Ltd., Otawara-shi, Tochigi 324-0036 (JP)
(72) Inventor: IMAI, Tsuneaki, Otawara-shi, Tochigi 324-0036 (JP)
(74) Representative: Henkel & Partner mbB
(86) International application number: PCT/JP2018/032403
(87) International publication number: WO 2019/176137

(57) **Abstract**

A radiation detection panel pertaining to an embodiment of the present invention is provided with: a substrate; a plurality of photoelectric conversion elements provided on one surface of the substrate; a light-transmitting insulating layer provided on the plurality of photoelectric conversion elements; a protective layer provided on at least the insulating layer; a light-transmitting junction layer provided between the insulating layer and the protective layer, the junction layer including a material having a reactive group for chemically bonding with an inorganic material and/or a reactive group for chemically bonding with an organic material; a scintillator for covering the plurality of photoelectric conversion elements, the scintillator being provided on the protective layer; and a moisture-proof layer for covering at least the scintillator.

## Description

### [Technical Field]

Embodiments described herein relate generally to a radiation detection panel, a radiation detector, and a method for manufacturing the radiation detection panel.

### [Background Art]

An X-ray detector is an example of the radiation detector. The X-ray detector is provided with a scintillator that converts X-rays into fluorescence and an array substrate that converts fluorescence into electrical signals. In addition, in order to improve the utilization efficiency of fluorescence and improve the sensitivity characteristic, a reflection part may be further provided on the scintillator.

The scintillator may be directly formed on the array substrate by using, for example, a vacuum vapor deposition method. However, since the scintillator is formed from a halogen compound such as CsI (cesium iodide): Tl (thallium) or CsI: Na (sodium), halogen reacts with a material of interconnect etc. when the scintillator is formed, so that there is a problem that corrosion easily occurs. For example, on the surface of the array substrate, in addition to a plurality of elements such as photodiodes or CMOS (Complementary Metal Oxide Semiconductor) sensors, interconnects for electrically connecting the plurality of elements is provided. Therefore, the interconnects or the like may be corroded when the scintillator is formed.

Therefore, a technique has been proposed in which a protective layer is formed on the surface of the array substrate and the scintillator is formed on the protective layer.

However, if the protective layer is simply formed on the surface of the array substrate, the protective layer may peel off from the surface of the array substrate when the scintillator is formed. When the protective layer is peeled off from the surface of the array substrate, a gap is generated between the scintillator and the array substrate, and the light traveling from the scintillator to the array substrate may be reflected or diffused in this gap. As a result, the efficiency of light transmission to the array substrate may decrease, and the brightness of the pixels of the X-ray image and the sharpness of the X-ray image may decrease. If the brightness of the pixels of the X-ray image or the sharpness of the X-ray image decreases, the image quality of the X-ray image may deteriorate.

Therefore, it was desired to develop a technology that can suppress peeling of the protective layer.

### [Citation List]

### [Patent Literature]

[Patent Literature 1] JP 2007-192807 A (Kokai)

### [Summary of Invention]

### [Problem to be Solved by Invention]

The problem to be solved by the invention is to provide a radiation detection panel, a radiation detector, and a method for manufacturing the radiation detection panel, which is able to suppress peeling of a protective layer.

### [Means for Solving Problem]

According to one embodiment, a radiation detection panel includes a substrate, a plurality of photoelectric conversion elements, an insulating layer, a protective layer, a bonding layer, a scintillator, and a moisture-proof layer. The photoelectric conversion elements are provided on one surface of the substrate. The insulating layer is provided on the photoelectric conversion elements and is light transmissive. The protective layer is provided at least on the insulating layer. The bonding layer is provided between the insulating layer and the protective layer, includes a material having at least one of a reactive group that chemically bonds to an inorganic material and a reactive group that chemically bonds to an organic material, and is light transmissive. The scintillator is provided on the protective layer and covers the plurality of photoelectric conversion elements. The moisture-proof layer covers at least the scintillator.

### [Brief Description of Drawings]

[FIG. 1]

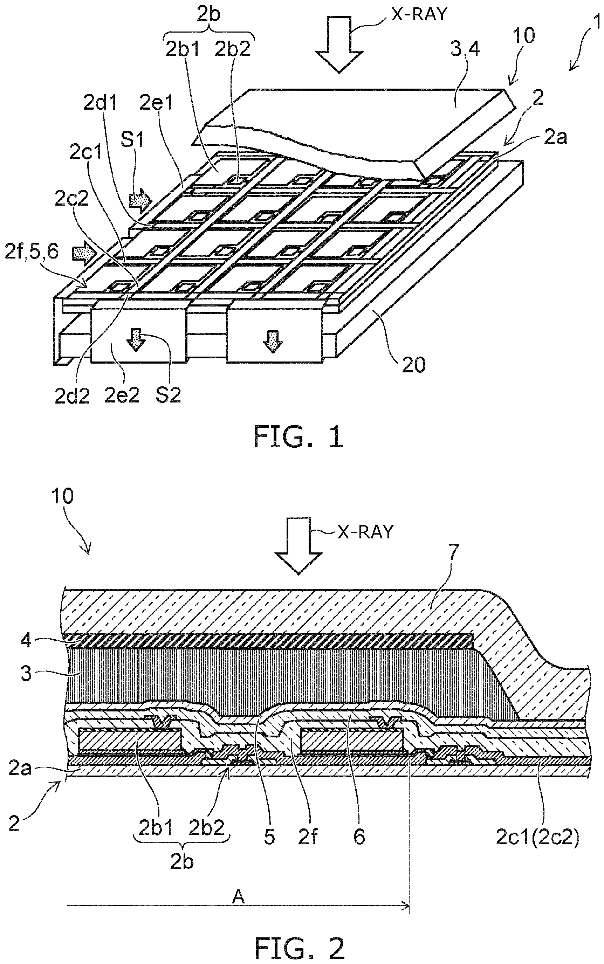

   FIG. 1 is a schematic perspective view for illustrating an X-ray detection panel and an X-ray detector according to the embodiment.
[FIG. 2]
   FIG. 2 is a schematic cross sectional view of the X-ray detection panel.
[FIG. 3]

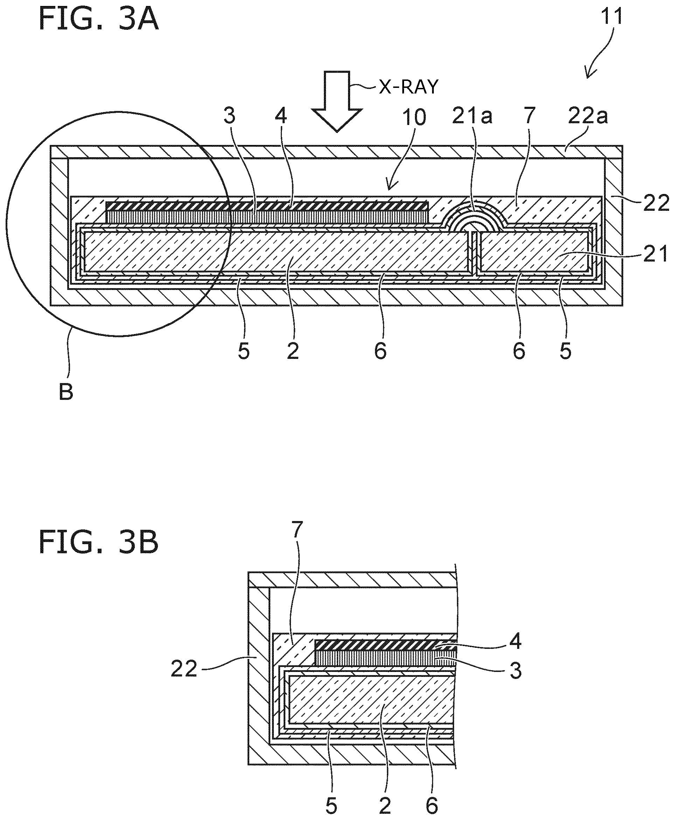

   FIG. 3A is a schematic cross sectional view for illustrating an X-ray detector according to other embodiment, FIG. 3B is a schematic enlarged view of a portion B in FIG. 3A.

### [Embodiments of Invention]

Various embodiments are described below with reference to the accompanying drawings. In the drawings, similar components are marked with like reference numerals, and a detailed description is omitted as appropriate.

Further, the radiation detector according to the embodiment of the invention can be applied to various radiations such as γ-rays as well as X-rays. Here, as an example, description will be given by taking a case relating to X-rays as a typical one of radiations. Therefore, by replacing "X-ray" in the following embodiments with "other radiation", it can be applied to other radiation.

Further, the radiation detector can be used, for example, in general medical care and dental care. However, the application of the radiation detector is not limited to these.

### (X-ray detection panel and X-ray detector)

FIG. 1 is a schematic perspective view for illustrating an X-ray detection panel 10 and an X-ray detector 1 according to the embodiment.

In order to avoid complexity, in FIG. 1, a moisture-proof layer 7 etc. is omitted.

FIG. 2 is a schematic cross sectional view of the X-ray detection panel 10.

The X-ray detector 1 serving as a radiation detector is an X-ray plane sensor which detects an X-ray image as a radiation image.

As shown in FIG. 1 and FIG. 2, the X-ray detector 1 is provided with the X-ray detection panel 10 and a circuit board 20.

The X-ray detection panel 10 is provided with an array substrate 2, a scintillator 3, a reflective layer 4, a protective layer 5, a bonding layer 6, and the moisture-proof layer 7.

The array substrate 2 converts fluorescence (visible light) converted from X-ray by the scintillator 3 to an electrical signal.

The array substrate 2 includes a substrate 2a, a photoelectric conversion part 2b, a control line (or gate line) 2c1, a data line (or signal line) 2c2, an interconnect pads 2d1, 2d2, an insulating layer 2f or the like.

The number of the photoelectric conversion part 2b, the control line 2c1, and the data line 2c2 or the like is not limited to the illustration.

The substrate 2a is plate-shaped, and formed of a light transmissive material such as non-alkaline glass.

A plurality of photoelectric conversion parts 2b are provided on one surface of the substrate 2a. The photoelectric conversion part 2b may have a rectangular shape. The photoelectric conversion part 2b is provided in each of a plurality of regions defined by a plurality of control lines 2c1 and a plurality of data lines 2c2 in a plan view. The plurality of photoelectric conversion parts 2b are arranged in a matrix. Note that one photoelectric conversion part 2b corresponds to one pixel in the X-ray image.

A photoelectric conversion element 2b1 and a thin film transistor (TFT) 2b2 that is a switching element are provided in each of the plurality of photoelectric conversion parts 2b.

Further, a storage capacitor to which the charges converted in the photoelectric conversion element 2b1 are supplied can be provided. The storage capacitor has, for example, a rectangular flat plate shape, and can be provided below the thin film transistor 2b2. However, the photoelectric conversion element 2b1 can also serve as a storage capacitor depending on the capacity of the photoelectric conversion element 2b1. In the following, the case where the photoelectric conversion element 2b1 also serves as a storage capacitor is illustrated as an example.

The photoelectric conversion element 2b1 can be, for example, a photodiode or a CMOS sensor.

The thin film transistor 2b2 performs switching of storage and release of electric charge in the photoelectric conversion element 2b1 which plays the role of a storage capacitor.

The thin film transistor 2b2 includes a gate electrode, a source electrode, and a drain electrode. The gate electrode of the thin film transistor 2b2 is electrically connected to the corresponding control line 2c1. The drain electrode of the thin film transistor 2b2 is electrically connected to the corresponding data line 2c2. The source electrode of the thin film transistor 2b2 is electrically connected to the corresponding photoelectric conversion element 2b1. The anode side of the photoelectric conversion element 2b1 is electrically connected to the corresponding bias line (not shown). When the bias line is not provided, the anode side of the photoelectric conversion element 2b1 is electrically connected to the ground instead of the bias line.

A plurality of control lines 2c1 are provided in parallel with each other at a predetermined interval. The control line 2c1 extends in the row direction, for example. One control line 2c1 is electrically connected to one of the plurality of interconnect pads 2d1 provided near the peripheral edge of the substrate 2a. One interconnect pad 2d1 is electrically connected to one of a plurality of interconnects provided on the flexible printed circuit board 2e1. The other ends of the plurality of interconnects provided on the flexible printed board 2e1 are electrically connected to the readout circuit provided on the circuit board 20.

A plurality of data lines 2c2 are provided in parallel with each other at a predetermined interval. The data line 2c2 extends, for example, in the column direction orthogonal to the row direction. One data line 2c2 is electrically connected to one of the plurality of interconnect pads 2d2 provided near the peripheral edge of the substrate 2a. One interconnect pad 2d2 is electrically connected to one of a plurality of interconnects provided on the flexible printed circuit board 2e2. The other ends of the plurality of interconnects provided on the flexible printed board 2e2 are electrically connected to the signal detection circuit provided on the circuit board 20.

The insulating layer 2f is provided on the photoelectric conversion part 2b, the control line 2c1, and the data line 2c2. The insulating layer 2f can be formed of a material having a light-transmitting property and an insulating property. The insulating layer 2f can be formed of, for example, an inorganic material such as silicon nitride (SiN). The insulating layer 2f can also be formed of an organic material such as acrylic resin, polyethylene, polypropylene, butyral, or the like.

The scintillator 3 is provided on the protective layer 5 and covers at least the plurality of photoelectric conversion elements 2b1. The scintillator 3 is provided so as to cover the effective pixel area A. The effective pixel area A is an area in which a plurality of photoelectric conversion elements 2b1 are provided on the substrate 2a.

The scintillator 3 converts incident X-rays into fluorescence.

The scintillator 3 includes a halogen compound as a main component. The scintillator 3 can be formed by using, for example, cesium iodide (CsI): thallium (TI), sodium iodide (NaI): thallium (Tl), or cesium bromide (CsBr): europium (Eu). The scintillator 3 can be formed by using, for example, a vacuum vapor deposition method. When the scintillator 3 is formed by using the vacuum evaporation method, the scintillator 3 composed of an aggregate of a plurality of columnar crystals is formed.

The reflective layer 4 is provided in order to improve the utilization efficiency of fluorescence and improve sensitivity characteristics. That is, the reflection layer 4 reflects, of the fluorescence generated in the scintillator 3, the light that is directed toward the side opposite to the side where the photoelectric conversion part 2b is provided, and directs the light toward the photoelectric conversion part 2b.

The reflective layer 4 can be, for example, a resin layer including particles having a light scattering property such as titanium oxide (TiO₂) or a metal layer including a metal such as aluminum.

The reflective layer 4 is not always necessary, and may be appropriately provided depending on the required sensitivity characteristics and the like.

The protective layer 5 is provided so as to cover the array substrate 2.

As mentioned above, the scintillator 3 is formed by using a halogen compound such as iodide or bromide. Therefore, when the scintillator 3 is formed, a part of the control line 2c1, a part of the data line 2c2, the interconnect pad 2d1, the interconnect pad 2d2, etc. exposed from the insulating layer 2f may be corroded. Therefore, the protective layer 5 is provided to suppress the occurrence of corrosion when the scintillator 3 is formed.

The protective layer 5 can be formed of, for example, a material having an insulating property, a water vapor blocking property, a light transmitting property, and a corrosion resistance against a substance included in the scintillator 3. The protective layer 5 can include, for example, an organic material such as acrylic resin, polyethylene, polypropylene, butyral, or polyparaxylylene. In this case, if the protective layer 5 including polyparaxylylene as the main component is used, the adhesion with the moisture-proof layer 7 including polyparaxylylene as the main component can be further improved. The protective layer 5 can also include, for example, a carbon crystal that is an inorganic material as a main component.

In general, the interconnect or the like to be protected by the protective layer 5 is provided on the side of the array substrate 2 where the scintillator 3 is provided. Therefore, the protective layer 5 may be provided on at least the side of the array substrate 2 on which the scintillator 3 is provided. For example, the protective layer 5 may be provided at least on the insulating layer 2f.

However, the protective layer 5 can be provided so as to cover the entire array substrate 2. In this way, the protective layer 5 can be easily formed. For example, if the array substrate 2 is placed inside a chamber where the material of the protective layer 5 is evaporated, the protective layer 5 that covers the entire array substrate 2 can be easily formed.

Further, the fluorescence generated in the scintillator 3 passes through the protective layer 5 and reaches the photoelectric conversion element 2b1. Therefore, the thickness of the protective layer 5 is preferably thin. The thickness of the protective layer 5 can be, for example, 15 µm or less.

Here, if the protective layer 5 is directly provided on the surface of the array substrate 2, the protective layer 5 may peel off from the surface of the array substrate 2. For example, when the protective layer 5 including an organic material is directly formed on the insulating layer 2f including an inorganic material, the bonding force between the protective layer 5 and the insulating layer 2f may be weakened. In such a case, when thermal stress or the like occurs during formation of the scintillator 3 or during operation of the X-ray detector 1, the protective layer 5 may peel off from the insulating layer 2f, and a gap may be generated between the protective layer 5 and the insulating layer 2f. That is, the gap may be generated between the scintillator 3 and the photoelectric conversion element 2b1. When such a gap is generated, the light traveling from the scintillator 3 to the photoelectric conversion element 2b1 may be reflected or diffused in this gap, and the efficiency of light transmission to the photoelectric conversion element 2b1 may be reduced. If the transmission efficiency of light to the photoelectric conversion element 2b1 decreases, the brightness of the pixels of the X-ray image and the sharpness of the X-ray image decrease, and the image quality of the X-ray image may deteriorate.

Therefore, in the X-ray detection panel 10 according to the embodiment, the bonding layer 6 is provided between the array substrate 2 (insulating layer 2f) and the protective layer 5.

The bonding layer 6 bonds the protective layer 5 and the array substrate 2. The bonding layer 6 can be formed of, for example, a material that is light transmissive and has at least one of a reactive group that chemically bonds to an inorganic material and a reactive group that chemically bonds to an organic material.

In this case, the bonding layer 6 can be formed of, for example, a silane coupling agent or the like. However, the material of the bonding layer 6 is not limited to the silane coupling agent.

For example, the insulating layer 2f may include an inorganic material (such as silicon nitride) and the protective layer 5 may include an organic material (such as polyparaxylylene). In such a case, the material of the bonding layer 6 may have a reactive group that chemically bonds to the inorganic material of the insulating layer 2f and a reactive group that chemically bonds to the organic material of the protective layer 5.

In addition, for example, the insulating layer 2f may include an organic material (for example, acrylic resin), and the protective layer 5 may include an organic material. In such a case, the material of the bonding layer 6 may have a reactive group that chemically bonds to the organic material of the insulating layer 2f and a reactive group that chemically bonds to the organic material of the protective layer 5.

For example, the insulating layer 2f may include an inorganic material and the protective layer 5 may include an inorganic material (for example, carbon crystal). In such a case, the material of the bonding layer 6 may have a reactive group that chemically bonds to the inorganic material of the insulating layer 2f and a reactive group that chemically bonds to the inorganic material of the protective layer 5.

Further, for example, the insulating layer 2f may include an organic material and the protective layer 5 may include an inorganic material. In such a case, the material of the bonding layer 6 may have a reactive group that chemically bonds to the organic material of the insulating layer 2f and a reactive group that chemically bonds to the inorganic material of the protective layer 5.

In this way, the protective layer 5 and the array substrate 2 (insulating layer 2f) are firmly bonded via the bonding layer 6 having a reactive group.

The bonding layer 6 may be provided at least between the scintillator 3 and the array substrate 2. For example, the bonding layer 6 can be provided in the effective pixel region A in a plan view (when viewed from the X-ray incident side). Further, the bonding layer 6 can also be provided in the region where the scintillator 3 is formed. If there is no gap in these regions, it is possible to prevent the efficiency of light transmission to the photoelectric conversion element 2b1 from decreasing.

The bonding layer 6 can be provided so as to cover the surface of the array substrate 2 on the side where the scintillator 3 is provided. The moisture-proof layer 7 is provided on the protective layer 5 on the side of the array substrate 2 on which the scintillator 3 is provided. Therefore, if the bonding layer 6 is provided so as to cover the surface of the array substrate 2 on the side where the scintillator 3 is provided, the bonding strength between the moisture-proof layer 7 and the array substrate 2 can be increased. Therefore, the moisture proof can be improved.

Further, the bonding layer 6 can be provided so as to cover the entire array substrate 2. In this way, the bonding layer 6 can be easily formed. For example, if the array substrate 2 is placed inside the chamber where the material of the bonding layer 6 is evaporated, the bonding layer 6 that covers the entire array substrate 2 can be easily formed.

Further, the fluorescence generated in the scintillator 3 passes through the bonding layer 6 and reaches the photoelectric conversion element 2b1. Therefore, it is preferable that the bonding layer 6 is thin. The thickness of the bonding layer 6 can be, for example, 15 µm or less.

If the bonding layer 6 is provided, it is possible to prevent the protective layer 5 from peeling off even if thermal stress or the like occurs during formation of the scintillator 3 or during operation of the X-ray detector 1. Therefore, it is possible to prevent a gap from being generated between the scintillator 3 and the photoelectric conversion element 2b1, and thus it is possible to suppress a decrease in light transmission efficiency to the photoelectric conversion element 2b1. As a result, it is possible to prevent the brightness of the pixels of the X-ray image and the sharpness of the X-ray image from deteriorating and deteriorating the image quality of the X-ray image.

The moisture-proof layer 7 is provided to prevent the characteristics of the reflective layer 4 and the characteristics of the scintillator 3 from being deteriorated by water vapor included in the air. In this case, if the scintillator 3 includes CsI: Tl, CsI: Na, or the like, the resolution characteristics may be significantly deteriorated due to humidity or the like.

Therefore, the moisture-proof layer 7 covers at least the scintillator 3. When the reflection layer 4 is provided, the moisture-proof layer 7 covers the scintillator 3 and the reflection layer 4.

The moisture-proof layer 7 can be provided so as to cover the entire array substrate 2 provided with the bonding layer 6, the protective layer 5, and the scintillator 3. In this way, the moisture-proof layer 7 can be easily formed. For example, if the array substrate 2 provided with the bonding layer 6, the protective layer 5, and the scintillator 3 is placed inside the chamber where the material of the moisture-proof layer 7 is evaporated, the moisture-proof layer 7 that covers all of them can be easily formed.

The moisture-proof layer 7 can be formed of, for example, a material having an insulating property, a water vapor blocking property, a light transmitting property, and a corrosion resistance against a substance contained in the scintillator 3. The moisture-proof layer 7 can include, for example, polyparaxylylene, which is an organic material, as a main component. The moisture-proof layer 7 can include, for example, a carbon crystal which is an inorganic material as a main component. In this case, if the material of the moisture-proof layer 7 is the same as the material of the protective layer 5, the adhesion between the moisture-proof layer 7 and the protective layer 5 can be improved. Therefore, the moisture resistance can be improved.

The circuit board 20 is provided on the side of the array substrate 2 opposite to the side of the scintillator 3.

The circuit board 20 is provided with a readout circuit and a signal detection circuit.

The readout circuit switches the thin film transistor 2b2 between the on state and the off state. The control signal S1 is input to the readout circuit from the image processing circuit or the like. The readout circuit inputs the control signal S1 to the control line 2c1 according to the scanning direction of the X-ray image. For example, the readout circuit sequentially inputs the control signal S1 to each control line 2c1 via the flexible printed board 2e1. The thin film transistor 2b2 is turned on by the control signal S1 input to the control line 2c1, and the electric charge (image data signal S2) from the photoelectric conversion element 2b1 serving as a storage capacitor can be received.

The signal detection circuit includes a plurality of integrating amplifiers, a selection circuit, an AD converter, and the like. One integrating amplifier is electrically connected to one data line 2c2. The integrating amplifier sequentially receives the image data signal S2 from the photoelectric conversion part 2b. Then, the integrating amplifier integrates the current flowing within a fixed time and outputs a voltage corresponding to the integrated value to the selection circuit. In this way, it becomes possible to convert the value of the current (charge amount) flowing through the data line 2c2 into the voltage value within a predetermined time. That is, the integrating amplifier converts the image data information corresponding to the intensity distribution of fluorescence generated in the scintillator 3 into potential information.

The selection circuit selects an integrating amplifier for reading out and sequentially reads out the image data signal S2 converted into potential information.

The AD converter sequentially converts the readout image data signal S2 into a digital signal. The image data signal S2 converted into a digital signal is input to the image processing circuit.

The image processing circuit forms an X-ray image based on the image data signal S2 converted into a digital signal. The image processing circuit may be provided on the circuit board 20 or may be provided outside the X-ray detector 1. When the image processing circuit is provided outside the X-ray detector 1, the communication can be performed wirelessly or can be performed via wiring.

In addition, a housing that houses the X-ray detection panel 10 can also be provided. The housing may have a box shape. The surface of the housing that faces the scintillator 3 can be formed of a material that transmits X-rays. Further, a support plate can be provided inside the housing. The X-ray detection panel 10 can be provided on the surface of the support plate on the side on which X-rays are incident. The circuit board 20 can be provided on the surface of the support plate opposite to the side on which the X-rays are incident.

FIG.3A is a schematic cross sectional view for illustrating an X-ray detector 11 according to other embodiment.

FIG. 3B is a schematic enlarged view of a portion B in FIG. 3A.

As shown in FIGS. 3A and 3B, the X-ray detector 11 is provided with the X-ray detection panel, a communication circuit board 21, and a housing 22.

In the embodiment, the circuit board 20 and the image processing circuit are provided outside the X-ray detector 11.

The communication circuit board 21 is electrically connected to the X-ray detection panel 10 via an interconnect 21a. The communication circuit board 21 performs communication between the X-ray detection panel 10 and the circuit board 20 and between the X-ray detection panel 10 and the image processing circuit. For example, the communication circuit board 21 receives the control signal S1 from the circuit board 20 and inputs the received control signal S1 to the predetermined control line 2c1. For example, the communication circuit board 21 receives the image data signal S2 from the X-ray detection panel 10 and transmits it to the circuit board 20. The communication circuit board 21 may be configured to communicate wirelessly, or may be configured to communicate via interconnects.

The communication circuit board 21, the circuit board 20, and the image processing circuit may be electrically connected to the X-ray detection panel 10, and these may be housed inside the housing 22.

That is, the X-ray detector can include at least one of the circuit board 20 electrically connected to the X-ray detection panel 10 or the communication circuit board 21.

The bonding layer 6 covers the entire array substrate 2, the communication circuit substrate 21, and the interconnect 21a. For example, if the X-ray detection panel 10 and the communication circuit board 21, which are electrically connected via the interconnect 21a, are placed inside the chamber where the material of the bonding layer 6 is evaporated, the bonding layer 6 that covers the entire of the array board 2, the communication circuit board 21 and the interconnect 21a can be easily formed.

The protective layer 5 covers the entire bonding layer 6. For example, if the array substrate 2, the communication circuit board 21, and the interconnect 21a covered with the bonding layer 6 are placed inside the chamber where the material of the protective layer 5 is evaporated, the protective layer 5 covering the entire bonding layer 6 can be easily formed.

The moisture-proof layer 7 covers the entire protective layer 5. For example, if the array substrate 2, the communication circuit board 21, and the interconnect 21a covered with the protective layer 5 are placed inside the chamber which the material of the moisture-proof layer 7 is evaporated, the moisture-proof layer 7 covering the entire protective layer 5 can be easily formed.

According to the embodiment, the array substrate 2, the communication circuit board 21, and the interconnect 21a can be integrated by the bonding layer 6, the protective layer 5, and the moisture-proof layer 7.

The housing 22 has a box shape with one end open. Inside the housing 22, the array substrate 2, the communication circuit board 21, and the interconnect 21a covered with the bonding layer 6, the protective layer 5, and the moisture-proof layer 7 are housed. If the array substrate 2, the communication circuit board 21, and the interconnect 21a are integrated by the bonding layer 6, the protective layer 5, and the moisture-proof layer 7, the housing 22 can be easily stored inside.

The housing 22 can also be formed by using, for example, an aluminum alloy, polyphenylene sulfide resin, polycarbonate resin, carbon fiber reinforced plastic (CFRP; Carbon-Fiber-Reinforced Plastic) or the like.

An entrance plate 22a closes the opening of the housing 22. The entrance plate 22a transmits X-rays. The entrance plate 22a is formed using a material having a low X-ray absorption rate. The entrance plate 22a can be formed using, for example, carbon fiber reinforced plastic.

### (X-ray detection panel and Method for manufacturing the X-ray detector)

Next, a method for manufacturing the X-ray detection panel 10 and the X-ray detector 1 according to the embodiment will be illustrated.

First, the photoelectric conversion part 2b, the control line 2c1, the data line 2c2, the interconnect pads 2d1 and 2d2, the insulating layer 2f, and the like are sequentially formed on the substrate 2a to form the array substrate 2. The array substrate 2 can be created using, for example, a semiconductor manufacturing process.

Next, the bonding layer 6 is formed on at least the effective pixel area A. The bonding layer 6 can also be formed on the region where the scintillator 3 is formed. The bonding layer 6 can also be formed on the surface of the array substrate 2 on the side where the scintillator 3 is provided. The bonding layer 6 can also be formed so as to cover the entire array substrate 2.

The bonding layer 6 can be formed, for example, by supplying a melted material to a predetermined area or the entire surface of the array substrate 2 and drying the material.

Next, the protective layer 5 is formed on the bonding layer 6. The protective layer 5 can be formed at least on the side of the array substrate 2 where the scintillator 3 is provided. The protective layer 5 can also be formed so as to cover the entire array substrate 2.

The protective layer 5 can be formed, for example, by placing the array substrate 2 having the bonding layer 6 formed therein inside the chamber where the material is evaporated. In this case, a mask or the like can be used to limit the region where the protective layer 5 is formed.

Next, the scintillator 3 is formed on at least the effective pixel area A. The scintillator 3 can be formed, for example, by forming a film of cesium iodide: thallium using a vacuum deposition method or the like. In this case, the thickness of the scintillator 3 can be about 600 µm.

When forming the scintillator 3, a mask is used so that the vapor deposition material does not reach the vicinity of the peripheral edge of the array substrate 2. Therefore, the peripheral region of the scintillator 3 becomes thinner toward the outside.

Next, the reflective layer 4 is formed on the scintillator 3. The reflective layer 4 can be formed, for example, by applying a resin including light-scattering particles made of titanium oxide (TiO₂) or the like onto the scintillator 3.

Next, the moisture-proof layer 7 is formed on at least the scintillator 3. When the reflective layer 4 is provided, the moisture-proof layer 7 is formed on the scintillator 3 and the reflective layer 4. The moisture-proof layer 7 can also be formed so as to cover the entire array substrate 2 on which the bonding layer 6, the protective layer 5, and the scintillator 3 are formed. The moisture-proof layer 7 can be formed, for example, by placing the array substrate 2 provided with the bonding layer 6, the protective layer 5, the scintillator 3, and the reflective layer 4 inside the chamber where the material is evaporated.

The X-ray detection panel 10 can be manufactured as described above.

As illustrated in FIG. 3A, the array substrate 2, the communication circuit board 21, and the interconnect 21a can be integrated by the bonding layer 6, the protective layer 5, and the moisture-proof layer 7.

Next, the X-ray detection panel 10 and the circuit board 20 are electrically connected via the flexible printed boards 2e1 and 2e2.

Next, the X-ray detection panel 10 and the circuit board 20 are housed inside the housing.

When the array substrate 2, the communication circuit board 21, and the interconnect 21a are integrated by the bonding layer 6, the protective layer 5, and the moisture-proof layer 7, these are housed inside the housing.

Then, if necessary, an electrical test, an X-ray image test, etc., for confirming whether the photoelectric conversion part 2b is abnormal or the electrical connection is abnormal.

The X-ray detectors 1 and 11 can be manufactured as described above.

As described above, the method of manufacturing the X-ray detection panel 10 according to the embodiment can include the following steps.

Forming the light transmissive insulating layer 2f on the plurality of photoelectric conversion elements 2b1.

Forming, on the insulating layer 2f, the bonding layer 6 including having at least one of a reactive group that chemically bonds to an inorganic material and a reactive group that chemically bonds to an organic material, the bonding layer being light transmissive.

Forming the protective layer 5 on the bonding layer 6.

Forming the scintillator 3 on the protective layer 5 so as to cover the plurality of photoelectric conversion elements 2b1. Forming the moisture-proof layer 7 that covers at least the scintillator 3.

Note that the details of each process can be the same as those described above, so a detailed description is omitted.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions. Moreover, above-mentioned embodiments can be combined mutually and can be carried out.

## Claims

1. A radiation detection panel (10), comprising:
a substrate (2a);
a plurality of photoelectric conversion elements (2b) provided on one surface of the substrate;
an insulating layer (2f) provided on the plurality of photoelectric conversion elements (2b), the insulating layer (2f) being light transmissive;
a protective layer (5) provided at least on the insulating layer (2f);
a bonding layer (6) provided between the insulating layer (2f) and the protective layer (5), the bonding layer (6) including a material having at least one of a reactive group that chemically bonds to an inorganic material and a reactive group that chemically bonds to an organic material, the bonding layer (6) being light transmissive;
a scintillator (3) provided on the protective layer (5) and covering the plurality of photoelectric conversion elements (2b); and
a moisture-proof layer (7) covering at least the scintillator (3).

2. The radiation detection panel (10) according to claim 1, wherein
the protective layer (5) and the moisture-proof layer (7) have insulating property, water vapor blocking property, light transmissivity, corrosion resistance to a substance included in the scintillator.

3. The radiation detection panel (10) according to claim 1 or 2, wherein
the protective layer (5) and the moisture-proof layer (7) include polyparaxylylene as a main component.

4. The radiation detection panel (10) according to claim 1 or 2, wherein
the protective layer (5) and the moisture-proof layer (7) include carbon crystal as a main component.

5. The radiation detection panel (10) according to one of claims 1 to 4, wherein
the scintillator (3) includes a halogen compound as a main component.

6. The radiation detection panel (10) according to claim 3 or 5, wherein
when the insulating layer (2f) includes an inorganic material,
the bonding layer (6) includes a material having a reaction group that chemically bonds to the inorganic material and a reactive group that chemically bonds to the polyparaxylylene.

7. The radiation detection panel (10) according to claim 3 or 5, wherein
when the insulating layer (2f) includes an organic material,
the bonding layer (6) includes a material having a reaction group that chemically bonds to the organic material and a reactive group that chemically bonds to the polyparaxylylene.

8. The radiation detection panel (10) according to claim 4 or 5, wherein
when the insulating layer (2f) includes an inorganic material,
the bonding layer (6) includes a material having a reaction group that chemically bonds to the inorganic material and a reactive group that chemically bonds to the carbon crystal.

9. The radiation detection panel (10) according to claim 4 or 5, wherein
when the insulating layer (2f) includes an organic material,
the bonding layer (6) includes a material having a reaction group that chemically bonds to the organic material and a reactive group that chemically bonds to the carbon crystal.

10. A radiation detector (1), comprising:
the radiation detection panel (10) according to one of claims 1 to 9; and
at least one of a circuit board (20) electrically connected to the radiation detection panel (10) or a communication circuit board (21).

11. A method for manufacturing the radiation detection panel (10) according to one of claims 1 to 9, the method comprising:
forming an insulating layer (2f) on a plurality of photoelectric conversion elements (2b), the insulating layer (2f) being light transmissive;
forming, on the insulating layer (2f), a bonding layer (6) including a material having at least one of a reactive group that chemically bonds to an inorganic material and a reactive group that chemically bonds to an organic material, the bonding layer (6) being light transmissive;
forming a protective layer (5) on the bonding layer (6);
forming a scintillator (3) on the protective layer (5), the scintillator (3) covering the plurality of photoelectric conversion elements (2b); and
forming a moisture-proof layer (7) covering at least the scintillator (3).
